# EUROPÄISCHE PATENTSCHRIFT

(11) **EP 0 691 879 B1**
(45) Veröffentlichungstag und Bekanntmachung des Hinweises auf die Patenterteilung: **25.06.1997**
(21) Anmeldenummer: 94912539.7
(22) Anmeldetag: 24.03.1994
(51) Int. Cl.: B01D 39/20, B01D 71/02, C04B 38/00, A61L 2/02, C02F 1/44

(54) **MIKROFILTERSCHEIBE UND VERFAHREN ZU IHRER HERSTELLUNG**
MICROFILTER PLATE AND PROCESS FOR MAKING IT
FILTRE MICROPOREUX SE PRESENTANT SOUS FORME DE DISQUE ET SON PROCEDE DE FABRICATION

(30) Priorität: 29.03.1993 EP 93105152
(43) Veröffentlichungstag der Anmeldung: 17.01.1996
(73) Patentinhaber: LUKOPAT AG, CH-8750 Glarus (CH)
(72) Erfinder: DILENGE, Angelo, D-56427 Siershan (DE); GRÜTER, Peter, CH-8105 Regensdorf (CH)
(74) Vertreter: Kahlhöfer, Hermann, Dipl.-Phys.
(86) Internationale Anmeldenummer: EP9400947
(87) Internationale Veröffentlichungsnummer: WO9422555

(56) Entgegenhaltungen:
- EP-A- 0 511 110
- DE-A- 3 926 411
- CHEMICAL ABSTRACTS, vol. 116, no. 2, 13. Januar 1992, Columbus, Ohio, US; abstract no. 10447d, Seite 240 ; & JP,A,3 215 373 (YAMAKAWA SANGYO KK) 20. September 1991

## Beschreibung

Die vorliegende Erfindung betrifft eine Mikrofilterscheibe, insbesondere zur Entkeimung von Wasser, und ein Verfahren zu ihrer Herstellung.

Bei der mechanischen Entkeimung von Wasser durch ein Mikrofilter kommt es darauf an, daß die Porengröße der Filtervorrichtung so klein ist, daß Bakterien mit einer typischen Größe von 0,4 bis 0,5 µm sicher zurückgehalten werden. Keramiken oder andere Materialien mit geeigneten Porengrößen sind bisher nicht in beliebigen Formen herstellbar, so daß großflächige Filter mit guter Filterwirkung nur mit großen Aufwand herzustellen und zu warten waren. Bisher verwendete großflächige Filterkerzen aus Keramik mit geeigneter Porengröße lassen sich nur schwer reinigen, wobei solche Filter im allgemeinen regelmäßig geöffnet und von Hand gesäubert werden müssen.

In der europäischen Patentanmeldung EP-A-0 542 678 (veröffentlicht am 19.5.93) auf die hier ausdrücklich Bezug genommen wird, ist eine selbstreinigende Filtereinrichtung beschrieben, die flache keramische Filterelemente enthält, wobei Reinigungseinheiten vorgesehen sind, die relativ zu den Filterflächen bewegbar und an diese unter Abarbeitung von Filtermaterial unter Druck anlegbar sind. Solche Filter lassen sich durch bewegliche und von außen angetriebene Reinigungsbürsten in zusammengebautem Zustand regelmäßig reinigen, wodurch sich die Wartung wesentlich vereinfacht.

Aufgabe der vorliegenden Erfindung ist es, eine großflächige flache Mikrofilterscheibe zur Verfügung zu stellen, welche z. B. in einer selbstreinigenden Filtereinrichtung eingesetzt werden kann. Außerdem soll ein geeignetes Verfahren zur Herstellung einer solchen Mikrofilterscheibe angegeben werden.

Zur Lösung dieser Aufgabe ist eine Mikrofilterscheibe geeignet, welche auf Kieselgurbasis hergestellt ist und mindestens zwei silikatisch gebundene Kieselgurarten enthält, von denen mindenstens eine vorkalzimiert und später gebrannt ist und wobei die Mikrofilterscheibe Porengrößen in der Größenordnung von 0,05 bis 0,5 µm, vorzugsweise 0,1 bis 0,3 µm aufweist. Es hat sich gezeigt, daß ein Anteil an vorkalziniertem Kieselgur bei der Herstellung die Stabilität der Mikrofilterscheibe gewährleistet und ein Gerüst für die Anordnung mindestens eines weiteren Anteils Kieselgur einer anderen Art bildet, wodurch sich erst eine stabile Form mit der gewünschten Porengröße ergibt. Insbesondere ist so eine monolithische runde Mikrofilterscheibe mit einem Durchmesser von mindestens 25 cm, vorzugsweise mindestens 35 cm, herstellbar. Eine solche Scheibe kann eine Dicke von 0,5 bis 2 cm, vorzugsweise etwa 1 bis 1,5 cm, aufweisen. Eine gute Filterwirkung bei gleichzeitiger Bruchfestigkeit ergibt sich etwa bis zu einer Dicke von 0,5 cm. Eine typische Durchlässigkeit einer erfindungsgemäßen Scheibe beträgt 0,5 bis 0,7 l/(min x dm²), vorzugsweise etwa 0,6.

Bevorzugt weist die Mikrofilterscheibe eine zentrale, vorzugsweise runde Öffnung auf, mit einem Durchmesser von 3 bis 15 cm, vorzugsweise 6 bis 10 cm. Mittels dieser Öffnung können solche Mikrofilterscheiben auf einer zentralen Säule gestapelt werden, wobei die Säule drehbar sein kann oder eine drehbare Achse enthalten kann, um eine Relativbewegung zwischen Mikrofilterscheiben und Reinigungsvorrichtungen zu ermöglichen.

Um solche Mikrofilterscheiben dicht in eine Filtervorrichtung einbauen zu können, müssen die Außen- und Innenränder der Mikrofilterscheibe maßhaltig geschnitten sein, was vorzugsweise mit einem Hochdruckwasserstrahlschneider erreichbar ist. Die Ränder werden bevorzugt mit einer Dichtung versehen, welche die Scheibe zumindest an einer Seite um 1 bis 8 mm, vorzugsweise 3 bis 5 mm, umgreift. Auf diese Weise kann die Scheibe sowohl seitlich wie gegenüber einer Unterlage dichtend montiert werden. Die Dichtungen an den Innen- und Außenseiten sollten etwa 1 - 3 mm dick sein, um geringfügige Größenänderungen der Scheibe ausgleichen zu können.

Um von Zeit zu Zeit eine automatische Reinigung der Oberfläche der Filterscheibe zu ermöglichen, weist diese bevorzugt eine offenporige Oberfläche auf, welche so weich ist, daß sie mittels einer Reinigungsbürste unter Erzielung eines Abriebes bearbeitet werden kann. Eine solche Reinigungsbürste kann beispielsweise Kunststoffborsten, insbesondere aus Nylon, oder Edelmetallborsten aufweisen, wobei zu einer Oberflächenreinigung in wenigen Arbeitsgängen ein Abrieb von 0,1 bis 0,5 µm erzielt werden soll. Dieser Abrieb soll nach ein bis zwanzig Relativbewegungen zwischen Oberfläche und Reinigungsbürste erreicht sein. Ein solcher Abrieb kann bei einem Spülvorgang anschließend ausgespült werden und gelangt nicht durch den Filter.

Zur Abtötung von eindringenden Keimen und zur Verhinderung eines Durchwachsens von auf der Oberfläche zurückgehaltenen Bakterien durch die Mikrofilterscheibe enthält diese bevorzugt nicht auswaschbare Anteile an Silbersalzen.

Eine Scheibe mit besonders großer Stabilität und guter Filterwirkung läßt sich durch die Verwendung von mindestens drei verschiedenen Kieselgurkomponenten und einem Anteil Kaolin erreichen. Dabei ist eine der Kieselgurkomponenten vorkalziniert und mindestens eine der Kieselgurkomponenten enthält besonders viele kleine Partikel, insbesondere etwa 15 % Partikel mit einer Größe unter 2 µm.

Bei einer bevorzugten Ausführungsform enthält die Mikrofilterscheibe 6 bis 12 Gewichtsprozent, vorzugsweise etwa 9 Gewichtsprozent, eines vorkalzinierten und gemeinsam mit den übrigen Bestandteilen nochmals gebrannten Kieselgurs, 25 bis 30 Gewichtsprozent Kaolin und als Rest mindestens eine weitere Sorte Kieselgur sowie gegebenenfalls Silbersalze und andere geringe herstellungsbedingte Bestandteile. Die Gewichtsprozente beziehen sich dabei auf die gebrannte trockene Scheibe.

Die Herstellung einer solchen Mikrofilterscheibe wird durch ein Verfahren mit folgenden Schritten gelöst:
a. Es werden gemischt: Mindestens 6 bis 12 Gewichtsteile, vorzugsweise etwa 9 Gewichtsteile, einer schon kalzinierten Sorte Kieselgur, mit 25 bis 35 Gewichtsteilen, vorzugsweise etwa 30 Gewichtsteilen, Kaolin und 53 bis 69 Gewichtsteile, vorzugsweise etwa 61 Gewichtsteile, mindestens einer nicht oder nur wenig kalzinierten Sorte Kieselgur;
b. Die Mischung wird angerührt in etwa 200 bis 250 Gewichtsteilen, vorzugsweise etwa 225 Gewichtsteilen, Wasser, unter Zugabe von 0,2 bis 0,35 Gewichtsteilen, vorzugsweise 0,27, mindestens eines Verflüssigungsmittels zur Herabsetzung der Oberflächenspannung und von 0,4 bis 0,8 Gewichtsteilen, vorzugsweise 0,5 bis 0,7, eines organischen Bindemittels;
c. Die Mischung wird mindestens so lange gerührt, bis sich eine deutliche Viskositätsänderung einstellt, vorzugsweise etwa 12 bis 15 Stunden;
d. Die fertig gerührte Mischung wird in eine Form aus Feuchtigkeit aufnehmenden Material, vorzugsweise Gips, gegossen;
e. Die Mischung wird langsam getrocknet, vorzugsweise über einen Zeitraum von 8 bis 25 Tagen, insbesondere 10 bis 20 Tagen;
f. Die getrocknete Mischung wird gebrannt, vorzugsweise bei einer Maximaltemperatur von 1000 bis 1050 ° C, insbesondere etwa 1035 ° C.

Wie anhand der Ausführungsbeispiele noch näher erläutert wird, bewirkt das Verflüssigungsmittel in Verbindung mit dem langen Rühren eine sehr gleichmäßige Verteilung der feinen Partikel der unterschiedlichen Kieselgursorten und des Kaolins. Das organische Bindemittel sorgt für einen Zusammenhalt der noch nicht gebrannten Scheibe beim Trocknen in und außerhalb der Form, während der schon kalzinierte Anteil Kieselgur ein stabiles Gerüst bildet und damit die Formstabilität (Grünfestigkeit) der noch nicht gebrannten Scheibe sichert. Durch den Anteil an kalzinierten Kieselgur stabilisiert und durch das organische Bindemittel zusammengehalten ordnen sich die übrigen Bestandteile sehr homogen so an, daß die gewünschte Porosität entsteht. Auf diese Weise ist es möglich, monolithische Scheiben mit bisher nicht erreichten Durchmessern und der gewünschten Porosität rißfrei herzustellen.

In einer bevorzugten Ausführungsform werden folgende Gewichtsanteile der Hauptbestandteile verwendet:
- etwa 40,72 eines ersten unkalzinierten oder wenig kalzinierten Kieselgurs,
- etwa 9,05 eines zweiten kalzinierten Kieselgurs,
- etwa 20,83 eines dritten unkalzinierten oder wenig kalzinierten Kieselgurs, und
- etwa 29,41 Kaolin.
Dabei werden Kieselgurarten unterschiedlicher Verteilung der Größe der Partikel eingesetzt, um insgesamt die gewünschte Mikroporosität in der Größenordnung von 0,05 bis 0,5 µm, vorzugsweise 0,1 bis 0,3 µm, zu erreichen.

Um zu verhindern, daß später an der Oberfläche zurückgehaltene Bakterien durch die Keramik hindurchwachsen können, werden der Mischung 0,2 bis 0,5 Gewichtsteile, vorzugsweise etwa 0,36, Silberoxyd zugegeben, welches ein solches Wachstum von Bakterien verhindert.

Für die rißfreie Herstellung der Mikrofilterscheiben ist die Wahl der Formen und die Art der Trocknung besonders wichtig. Bevorzugt wird daher eine Form aus einem geringfügig feuchten Gips angewendet, in welche die fertige Mischung gefüllt wird. Gips ist ein Material, welche saugfähig für Wasser ist, wobei völlig trockener Gips der Mischung so schnell Wasser entziehen würde, daß es zu einer Rißbildung kommen könnte. Daher werden geringfügig feuchte Gipsformen bevorzugt.

Um Scheiben mit einer Öffnung in der Mitte herzustellen, muß die Form einen zentralen Kern aufweisen, damit diese Öffnung direkt ausgespart bleibt. Da auch in diesem Bereich eine Rißbildung besonders leicht auftritt, sollte der Kern aus einem elastischen und saugfähigen Material bestehen, welches Verformungen der Scheibe beim Trocknen nachgibt, jedoch als Form stabil genug ist. Besonders geeignet ist ein Hohlzylinder aus Papier oder Pappe, welcher zunächst auch Feuchtigkeit aufsaugt, sich beim späteren Trocknen geringfügig verformen kann und schließlich leicht abzulösen ist.

Beim Brennen einer so vorgefertigten und vorgetrockneten Scheibe entsteht im allgemeinen eine verdichtete Oberfläche, welche für den vorgesehenen Zweck, nämlich die Filterung von Flüssigkeiten, zu undurchlässig und daher ungeeignet ist. Die Oberfläche wird daher nach dem Brennen mit einem abtragenden Werkzeug von der verdichteten Deckschicht befreit, wobei diese im allgemeinen eine Dicke von etwa 5 µm hat.

Schließlich kann die Scheibe maßgenau geschnitten werden, was bevorzugt mit einem Hochdruckwasserstrahlschneider erfolgt. Um die Scheibe dichtend in eine Filtervorrichtung einsetzen zu können, werden der Außen- und Innenrand der Platte mit einer die Ränder zumindest auf einer Seite der Platte umgreifenden Dichtschicht versehen. Die Dichtschicht kann vorzugsweise aus einem Material auf Silikonbasis bestehen und an der Platte festgeklebt werden.

Im folgenden werden anhand der Zeichnung und anhand besonderer Ausführungsbeispiele, auf die die Erfindung jedoch nicht beschränkt ist, weitere Einzelheiten, Vorteile und Modifikationen der Erfindung beschrieben.
- Fig. 1: zeigt eine Ansicht von oben auf eine erfindungsgemäße Scheibe,
- Fig. 2: einen Querschnitt durch eine solche Scheibe und
- Fig. 3: eine zur Herstellung einer solchen Scheibe geeignete Form.

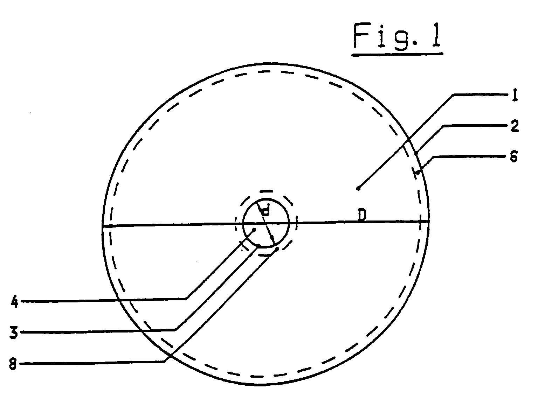

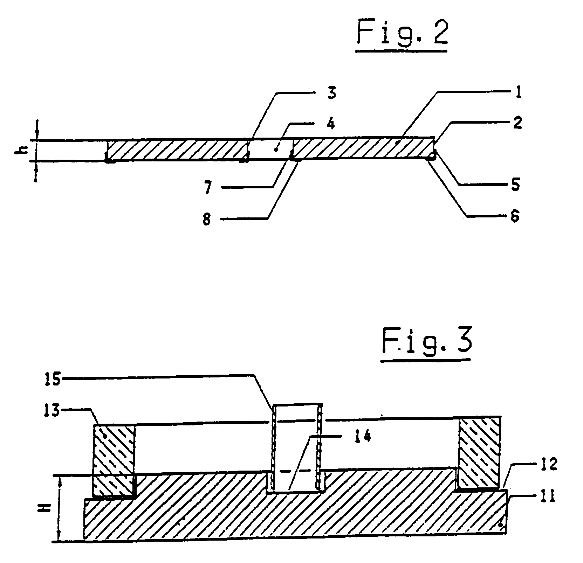

Die in den Figuren 1 und 2 dargestellte Ausführungsform einer Scheibe 1 hat einen Außendurchmesser D von etwa 40 cm und einen Innendurchmesser d der inneren Öffnung 4 von etwa 6,5 cm. Wie aus Figur 2 zu ersehen ist, ist der Außenrand 2 der Scheibe 1 mit einer Dichtung 5 versehen, welche mit einem Abschnitt 6 auch die Unterseite der Scheibe 1 umfaßt. Ebenso ist der Innenrand 3 der Scheibe 1 mit einer Dichtung 7 versehen, welche in einem Abschnitt 8 ebenfalls die Unterseite der Scheibe 1 umfaßt. Die Dichtabschnitte 6, 8 an der Unterseite der Scheibe 1 ermöglichen das abdichtende Auflegen auf eine die Scheibe 1 tragende Struktur, während die an dem Außenrand 2 und dem Innenrand 3 angebrachten Dichtungen 5, 6, zur Abdichtung gegenüber einer nicht dargestellten Behälterwand bzw. einer inneren zylindrischen Säule und zur Aufnahme geringfügiger Dehnungen der Scheibe unter Temperatureinfluß dienen. Die Dichtungen 5, 7 an dem Außen- bzw. Innenrand können auch getrennt von den Dichtungen 6, 8 an der einen Seite der Scheibe aufgebracht, vorzugsweise aufgeklebt, sein.

Die in Figur 3 schematisch dargestellte Form dient zur Herstellung einer erfindungsgemäßen Scheibe. Eine Grundplatte 11 mit einer Höhe H von etwa 50 bis 70 mm weist eine äußere Ringnut 12 auf, in welche ein ringförmiges Formteil 13 eingesetzt ist. Die Grundplatte 11 sollte eine Höhe H von 1/6 bis 1/10, vorzugsweise 1/8 des Durchmessers D der Scheibe 1 haben. Grundplatte 11 und ringförmiges Formteil 13 bestehen vorzugsweise aus Gips, welcher beim Füllen der Form nicht vollständig trocken sein soll. Die Grundplatte 11 weist außerdem eine kreis- oder ringförmige Vertiefung 14 in der Mitte auf, in welche ein hohlzylinderförmiger Kern 15, vorzugsweise aus Papier oder Pappe eingesetzt wird. In die durch Grundplatte 11, ringförmiges Formteil 13 und zylinderförmigen Einsatz 15 gebildete Form kann die zur Herstellung einer Scheibe 1 benötigte keramische Masse gegossen werden. Die Form saugt einen Teil der Flüssigkeit aus der abgegossenen Masse langsam auf und unterstützt so einen langsamen Trockungsprozeß. Um ein Verkleben der abgegossenen Masse mit der Gipsform zu vermeiden, kann diese mit einem Gleitmittel, insbesondere Talkum, bestrichen werden. Nach zwei bis fünf Stunden können die geformten Scheiben aus der Form genommen und auf einer glatten, saugfähigen Unterlage, vorzugsweise wiederum einer Gipsplatte, zum Trocknen ausgelegt werden. Um Risse zu vermeiden sollte die Trocknung bei Temperaturen zwischen 30 und 50 ° C, vorzugsweise 35 bis 40 ° C langsam erfolgen, so daß der Trocknungsverlauf etwa zwei bis drei Wochen dauern kann.

Nach der Trocknung erfolgt das Brennen der Scheibe, wobei bei einer Gesamtbranntdauer von 10 bis 15 Stunden eine Maximaltemperatur von etwa 1035 ° C für etwa 20 Minuten gehalten wird.

Im folgenden seien nochmals die Zutaten für die keramische Masse und das Verfahren bei ihrer Mischung anhand eines Ausführungsbeispiels, welches als besonders günstig angesehen wird, beschrieben.

In eine Vorlage von 4,4 l Wasser werden unter ständigem Rühren 6,5 g eines Verflüssigungsmittels, welches die Oberflächenspannung herabsetzt, und 12 g eines organischen Bindemittels zugegeben. Anschließend werden 900 g von einem ersten Kieselgur, 200 g von einem zweiten, vorkalzinierten Kieselgur, 460 g von einem dritten Kieselgur und 650 g Kaolin zugegeben. Nach intensivem Rühren werden außerdem 8 g Silberoxid beigemischt. Zur völligen Homogenisierung der gesamten Mischung wird diese ca. 15 bis 18 Stunden weitergerührt, wobei die Mischung abgußbereit ist, wenn sich die Viskosität fast sprunghaft um 10 bis 20 % verringert hat, d.h. die Mischung dünnflüssiger geworden ist. Der PH-Wert beim Ausgießen sollte bei ca. 9 liegen.

Die einzelnen Komponenten, insbesondere das Verflüssigungsmittel, das organische Bindemittel, können ihrerseits in Wasser gelöst zugegeben werden, wobei insgesamt für die Mischung eine Menge von 5 l Wasser erreicht werden soll.

Die folgende Tabelle enthält Angaben über die verwendeten unterschiedlichen Arten von Kieselgur bezüglich ihrer Zusammensetzung und ihrer Partikelgröße. Kieselgur, auch Diatomeerde genannt, ist ein natürlich vorkommendes Material, welches durch geologische Prozesse aus Kieselalgen entstanden ist. Solche Kieselgure sind unter dem Markennamen "Celite" von der Manville International Corporation, Denver, USA, erhältlich und werden mit den entsprechenden Zusammensetzung und sehr feinkörnig angeboten.

| Eigenschaft | Kieselgur 1 | Kieselgur 2 | Kieselgur 3 |
|---|---|---|---|
| Vorkalziniert | nein | ja | nein |
| Si 0₂ | 85,8 | 85,8 | 89,3 |
| Al₂ 0₃ | 3,8 | 3,8 | 4,2 |
| Fe₂ 0₃ | 1,2 | 1,2 | 1,4 |
| Ti 0₂ | 0,2 | 0,2 | 0,2 |
| Ca 0 | 0,5 | 0,5 | 0,6 |
| Mg 0 | 0,6 | 0,6 | 0,6 |
| Na₂ 0 oder K₂ 0 | 1,1 | 1,1 | 3,5 |
| P₂ 0 | 0,2 | 0,2 | - |

Erfindungsgemäße Scheiben eignen sich in besonderer Weise aufgrund ihrer monolithischen flachen Form für selbstreinigende Filtereinrichtungen, wie sie in der europäischen Patentanmeldung EP-A-0 542 678 (veröffentlicht am 19.5.93) beschrieben werden. Auf diese Anmeldung wird zur Vermeidung von Wiederholungen vollinhaltlich bezug genommen. Gemäß der dort beschriebenen Anwendungsform werden zwei Filterscheiben durch einen Stützrahmen gegeneinander abgestützt als Filterelement verwendet, welches von außen mit ungereinigtem Wasser beaufschlagt wird, welches innen gereinigt wieder abgezogen werden kann. Viele solche Filterscheiben übereinandergestapelt bilden einen Filter mit großer Oberfläche und damit großem Durchfluß pro Zeiteinheit. Die flache Form der Scheiben ermöglicht die Reinigung durch rotierende Bürsten, welche an einer Mittelachse befestigt sind, die durch die innere Öffnung jeder Scheibe hindurchgeführt ist. Von den Scheiben werden bei jeder Reinigung dünne Oberflächenschichten abgetragen, wodurch sich die ursprünglichen Eigenschaften der frischen Filterplatte wiedereinstellen. Mittels Ultraschall oder anderer Überwachungstechniken kann festgestellt werden, wenn die Scheiben bis zu einer Dicke von beispielsweise etwa 0,5 cm abgetragen sind. In diesem Falle besteht bei weiterer Benutzung Bruchgefahr, so daß die Scheiben ausgetauscht werden müssen. Die erfindungsgemäßen Scheiben sind aufgrund ihrer monolithischen Gestalt auch einfach auszuwechseln und erfordern keine komplizierten Strukturen zu ihrer gegenseitigen Abstützung und Abdichtung. Sie eignen sich daher in besonderer Weise für einen wirtschaftlichen Betrieb von Filtereinrichtungen zur Entkeimung von Wasser. Die besonders kleinen Poren ermöglichen eine sichere mechanische Zurückhaltung aller Bakterien. Zur geschmacklichen Aufbereitung kann gegebenenfalls noch ein Aktivkohlefilter nachgeschaltet werden. Auch können übliche Entkeimungsmittel auf der "Reinseite" der Filterplatten zwischen diesen angeordnet werden.

## Patentansprüche

1. Mikrofilterscheibe (1) auf Kieselgurbasis, insbesondere zur Entkeimung von Wasser, mit folgenden Merkmalen:
a. die Mikrofilterscheibe (1) enthält Anteile von mindestens zwei silicatisch gebundenen Kieselgurarten, von denen mindestens eine vorkalziniert und später gebrannt ist;
b. die Mikrofilterscheibe (1) weist Porengrößen in der Größenordnung von 0.05 bis 0,5 µm, vorzugsweise 0,1 bis 0,3 µm, auf.

2. Mikrofilterscheibe (1) nach Anspruch 1, dadurch gekennzeichnet, daß die Mikrofilterscheibe (1) monolithisch und rund ist und einen Durchmesser (D) von mindestens 25 cm, vorzugsweise mindestens 35 cm, hat.

3. Mikrofilterscheibe (1) nach Anspruch 1 oder 2, dadurch gekennzeichnet, daß die Mikrofilterscheibe (1) eine Dicke (h) von 0,5 bis 2 cm, vorzugsweise etwa 1 bis 1,5 cm, aufweist.

4. Mikrofilterscheibe (1) nach einem der Ansprüche 1 bis 3, dadurch gekennzeichnet, daß die Mikrofilterscheibe (1) eine zentrale, vorzugsweise runde Öffnung (4) aufweist, mit einem Durchmesser (d) von 3 bis 15 cm, vorzugsweise 6 bis 8 cm.

5. Mikrofilterscheibe (1) nach einem der vorhergehenden Ansprüche, dadurch gekennzeichnet, daß die Außen- (2) und Innenränder (3) der Mikrofilterscheibe (1) maßhaltig geschnitten sind, vorzugsweise mit einem Hochdruckwasserstrahlschneider.

6. Mikrofilterscheibe (1) nach einem der vorhergehenden Ansprüche, dadurch gekennzeichnet, daß die Außen- (2) und Innenränder (3) der Mikrofilterscheibe (1) mit Dichtungen (5, 6, 7, 8) versehen sind, welche die Scheibe an einer Seite um 1 bis 8 mm, vorzugsweise 3 bis 5 mm umgreifen.

7. Mikrofilterscheibe (1) nach einem der vorhergehenden Ansprüche, dadurch gekennzeichnet, daß die Scheibe eine offenporige Oberfläche aufweist, welche so weich ist, daß mittels einer Reinigungsbürste mit Nylonborsten, Edelmetallborsten oder dergleichen ein Abrieb von 0,1 bis 0,5 µm in wenigen Arbeitsgängen erzielbar ist.

8. Mikrofilterscheibe (1) nach einem der vorhergehenden Ansprüche, dadurch gekennzeichnet, daß die Scheibe nicht auswaschbare Anteile an Silbersalzen enthält.

9. Mikrofilterscheibe (1) nach einem der vorhergehenden Ansprüche, dadurch gekennzeichnet, daß die Scheibe mindestens drei Kieselgurkomponenten und einen Anteil Kaolin enthält.

10. Mikrofilterscheibe (1) nach einem der vorhergehenden Ansprüche, dadurch gekennzeichnet, daß die Scheibe etwa 6 bis 12 Gewichtsprozent (bezogen auf die trockene Scheibe), vorzugsweise etwa 9 Gewichtsprozent, vorkalzinierten und gemeinsam mit den übrigen Bestandteilen nochmals gebrannten Kieselgur, 25 bis 30 Gewichtsprozent Kaolin und als Rest mindestens eine weitere Sorte Kieselgur enthält und gegebenenfalls Silbersalze und herstellungsbedingte geringe andere Bestandteile.

11. Verfahren zur Herstellung einer Mikrofilterscheibe (1) mit Porengrößen von 0.05 bis 0,5 µm, vorzugsweise 0,1 bis 0,3 µm, und mit einem Durchmesser (D) größer als 25 cm, vorzugsweise größer als 35 cm, insbesondere einer Filterscheibe nach einem der Ansprüche 1 bis 10, mit folgenden Schritten:
a. es werden gemischt: mindestens 6 bis 12 Gewichtsteile, vorzugsweise etwa 9, einer schon kalzinierten Sorte Kieselgur, mit 25 bis 35 Gewichtsteilen, vorzugsweise etwa 30, Kaolin und 53 bis 69 Gewichtsteile, vorzugsweise etwa 61, mindestens einer nicht oder nur wenig kalzinierten Sorte Kieselgur;
b. die Mischung wird angerührt in etwa 200 bis 250 Gewichtsteilen, vorzugsweise etwa 225, Wasser unter Zugabe von 0,2 bis 0,35 Gewichtsteilen, vorzugsweise 0,27, mindestens eines Verflüssigungsmittels und von 0,4 bis 0,8 Gewichtsteilen, vorzugsweise 0,5 bis 0,7, eines organischen Bindemittels;
c. die Mischung wird mindestens so lange gerührt, bis sich eine deutliche Viskositätsänderung einstellt, vorzugsweise etwa 12 bis 15 Stunden;
d. die fertiggerührte Mischung wird in eine Form (11, 13, 15) aus feuchtigkeitsaufnehmendem Material, vorzugsweise Gips, gegossen;
e. die Mischung wird langsam getrocknet, vorzugsweise über einen Zeitraum von 8 bis 25 Tagen, insbesondere 10 bis 20 Tagen;
f. die getrocknete Mischung wird gebrannt, vorzugsweise bei einer Maximaltemperatur von 1000 bis 1050 °C, insbesondere 1035 °C.

12. Verfahren nach Anspruch 11, dadurch gekennzeichnet, daß folgende Gewichtsteile verwendet werden:
etwa 40,72 eines ersten unkalzinierten oder wenig kalzinierten Kieselgurs,
etwa 9.05 eines zweiten kalzinierten Kieselgurs,
etwa 20,82 eines dritten unkalzinierten oder wenig kalzinierten Kieselgurs und
etwa 29,41 Kaolin.

13. Verfahren nach einem der Ansprüche 11 oder 12, dadurch gekennzeichnet, daß der Mischung 0,2 bis 0,5 Gewichtsteile, vorzugsweise etwa 0,36, Silberoxid zugegeben werden.

14. Verfahren nach einem der Ansprüche 11, 12 oder 13, dadurch gekennzeichnet, daß die Form (11, 13) aus einem geringfügig feuchten Gips besteht.

15. Verfahren nach einem der Ansprüche 11 bis 14, dadurch gekennzeichnet, daß die Form (11, 13, 15) in der Mitte (14) mit einem zentralen Kern (15) aus einem elastischen und saugfähigen Material versehen wird, vorzugsweise einem Hohlzylinder aus Papier oder Pappe.

16. Verfahren nach einem der Ansprüche 11 bis 15, dadurch gekennzeichnet, daß nach dem Brennen die Oberfläche der Scheibe mit einem abtragenden Werkzeug von einer verdichteten Deckschicht mit einer Dicke von etwa 5 µm befreit wird.

17. Verfahren nach einem der Ansprüche 11 bis 16, dadurch gekennzeichnet, daß die gebrannte Mikrofilterscheibe (1) mit einem Hochdruckwasserstrahlschneider maßgenau geschnitten wird.

18. Verfahren nach einem der Ansprüche 11 bis 17, dadurch gekennzeichnet, daß der Außen- und Innenrand der Platte mit die Ränder zumindest auf einer Seite umgreifenden Dichtungen (5, 6, 7, 8) versehen werden, vorzugsweise auf Silikonbasis.

## Claims

1. Micro-filter disc (1) based on kieselguhr, especially for the sterilization of water having the following characteristics:
a) the micro-filter disc (1) contains at least two types of silicately bonded kieselguhr at least one of which is pre-calcined and burnt later;
b) the micro-filter disc (1) exhibits pore sizes of a magnitude of 0.05 to 0.5µm, preferably 0.1 to 0.3µm.

2. Micro-filter disc (1) according to claim 1, characterized in that the micro-filter disc (1) is monolithioal, is round, and has a diameter (D) of at least 25cm, preferably at least 35cm.

3. Micro-filter disc (1) according to claims 1 or 2 characterized in that the micro-filter disc (1) exhibits a thickness (h) of 0.5 to 2cm, preferably 1 to 1.5cm.

4. Micro-filter disc (1) according to claims 1 to 3, characterized in that the micro-filter disc (1) has a central, preferably round opening (4), having a diameter (d) of 3-15cm, preferably 6 to 8cm.

5. Micro-filter disc (1) according to one of the preceding claims, characterized in that the outer (2) and inner (3) margins of the micro-disc filter have been cut to measure, preferably by a high pressure water-jet cutter.

6. Micro-disc filter (1) according to one of the preceding claims, characterized in that the outer (2) and inner margins (3) of the micro-filter disc (1) have been provided with seals (5,6,7,8) which overlap the disc to one side by 1 to 8mm, preferably 3 to 5mm.

7. Micro-filter disc (1) according to one of the preceding claims, characterized in that the disc exhibits an open pore upper surface which is soft to an extent that cleaning brushes having NYLON bristles, precious metal bristles, or such, can scrape off from 0.1 to 0.5µm obtainable in a few working cycles.

8. Micro-filter disc (1) according to one of the preceding claims, characterized in that the disc contains portions of non-washable silver salts.

9. Micro-filter disc (1) according to one of the preceding claims, characterized in that the disc contains at least three components of kieselguhr and a portion of kaolin.

10. Micro-filter disc (1) according to one of the preceding claims, characterized in that the disc contains about 6 to 12 percent by weight, preferably about 9 percent by weight, pre-calcined and together with the remaining components again burnt kieselguhr, 25 to 30 percent by weight kaolin, and as a remainder a further kind of kieselguhr, silver salts and production mandated other components, the percent by weight indication is relative to the dried disc.

11. Method for the production of a micro-filter disc (1) having pore sizes of 0.05 to 0.5µm, preferably 0.1 to 0.3µm and a diameter (D) greater than 25cm, preferably greater than 35cm, especially a filter disc according to one of the claims 1-10, having the following steps:
a) it is being mixed: at least 6 to 12 parts by weight, preferably 9, an already calcined kind of kieselguhr, with 25 to 35 parts by weight, preferably about 30 kaolin, and 53 to 69 parts by weight, preferably about 61, of a non - or least very little calcined kind of kieselguhr;
b) the mixture is stirred into about 200 to 250 parts by weight, preferably about 225, of water while adding about 0.2 to 0.35 parts by weight, preferably 0.27, at least a liquefying agent and from 0.4 to 0.8 parts by weight, preferably 0.5 to 0.7, an organic binding agent;
c) stirring the mixture for a period of time of preferably 12 to 15 hours until a definite change in viscosity occurs;
d) pouring the ready-stirred mixture into a mold (11, 13, 15) made of moisture absorbing material, preferably plaster of paris;
e) slowly drying the mixture over a period of time of 8 to 25 days, particularly 10 to 20 days;
f) removing the dried mixture from the mold and thereafter burning the mixture under a temperature of 1000 to 1050°C, particularly 1035°C.

12. Method according to claim 11, characterized in that the following parts by weight are being used:
about 40.72 of a first non-calcined or little calcined kieselguhr,
about 9.05 of a second calcined kieselguhr,
about 20,82 of a third non-calcined or little calcined kieselguhr, and
about 29.41 kaolin.

13. Method according to claims 11 or 12, characterized in that the mixture is added 0.2 to 0.5 parts by weight, preferably about 0.36, of silver oxide.

14. Method according to one of the claims 11, 12 or 13 characterized in that the mold consists of a minimal moist plaster of paris.

15. Method according to one of the claims 11-14, characterized in that the mold (11,13,15) is being provided at its center (140 with a central core (15) consisting of an elastic and absorbent material, preferably a hollow cylinder of paper or card board.

16. Method according to one of the claims 11-15, characterized in that after burning, the upper surface of the disc is liberated from a denser cover layer having a thickness of about 5µm by a scraping tool.

17. Method according to one of the claims 11-16, characterized in that the burnt micro-filter disc is being cut to precise measurements by a high pressure water-jet.

18. Method according to one of the claims 11 to 17 characterized in that the outer and inner margins of the disc is provided with seals (5,6,7,8) overlapping to one side of the disc, preferably based on silicone.

## Revendications

1. Plaque filtrante microporeuse (1) à base de diatomite, notamment pour la stérilisation d'eau, comportant les caractéristiques suivantes :
a. la plaque filtrante microporeuse (1) contient des parts d'au moins deux sortes de diatomites, liées au silicate, parmi lesquelles au moins l'une est calcinée au préalable et cuite ensuite ;
b. la plaque filtrante microporeuse (1) présente des grosseurs de pores comprises entre 0,05 et 0,5 µm, de préférence entre 0,1 et 0,3 µm.

2. Plaque filtrante microporeuse (1) selon la revendication 1, caractérisée en ce que la plaque filtrante microporeuse (1) est monolithique et ronde et a un diamètre (D) de 25 cm au moins, de préférence 35 cm au moins.

3. Plaque filtrante microporeuse (1) selon la revendication 1 ou 2, caractérisée en ce que la plaque filtrante microporeuse (1) a une épaisseur (h) de 0,5 à 2 cm, de préférence de 1 à 1,5 cm environ.

4. Plaque filtrante microporeuse (1) selon l'une des revendications 1 à 3, caractérisée en ce que la plaque filtrante microporeuse (1) comporte un trou (4) central, rond de préférence, ayant un diamètre (d) de 3 à 15 cm, de préférence 6 à 8 cm.

5. Plaque filtrante microporeuse (1) selon l'une des revendications précédentes, caractérisée en ce que le bord extérieur (2) et le bord intérieur (3) de la plaque filtrante microporeuse (1) sont coupés aux dimensions exactes, de préférence au moyen d'un appareil à découper par jet d'eau à haute pression.

6. Plaque filtrante microporeuse (1) selon l'une des revendications précédentes, caractérisée en ce que le bord extérieur (2) et le bord intérieur (3) de la plaque filtrante microporeuse (1) sont munis de joints (5, 6, 7, 8) qui font le tour de la plaque en en recouvrant un côté sur 1 à 8 mm, de préférence sur 3 à 5 mm.

7. Plaque filtrante microporeuse (1) selon l'une des revendications précédentes, caractérisée en ce que la plaque a une surface à pores ouverts qui est suffisamment tendre pour qu'on puisse obtenir une abrasion de 0,1 à 0,5 µm en quelques opérations au moyen d'une brosse à poils de nylon, de métal précieux ou analogues.

8. Plaque filtrante microporeuse (1) selon l'une des revendications précédentes, caractérisée en ce que la plaque contient des parts de sels d'argent qui ne peuvent pas être enlevées par lavage.

9. Plaque filtrante microporeuse (1) selon l'une des revendications précédentes, caractérisée en ce que la plaque contient au moins trois composants de diatomite et une part de kaolin.

10. Plaque filtrante microporeuse (1) selon l'une des revendications précédentes, caractérisée en ce que la plaque contient environ 6 à 12 % en poids (rapporté à la plaque sèche), de préférence environ 9 % en poids, de diatomite calcinée au préalable et encore cuite avec les autres constituants, 25 à 30 % en poids de kaolin et, pour le reste, au moins une autre sorte de diatomite, et éventuellement des sels d'argent et d'autres constituants en faible part et nécessaires à la fabrication.

11. Procédé de fabrication d'une plaque filtrante microporeuse (1) avec des grosseurs de pores comprises entre 0,05 et 0,5 µm, de préférence entre 0,1 et 0,3 µm, et avec un diamètre (D) supérieur à 25 cm, de préférence supérieur à 35 cm, notamment une plaque filtrante selon l'une des revendications 1 à 10, avec les étapes suivantes :
a. on mélange : au moins 6 à 12 parts en poids, de préférence environ 9, d'une sorte de diatomite déjà calcinée, avec 25 à 35 parts en poids, de préférence environ 30, de kaolin et avec 53 à 69 parts en poids, de préférence environ 61, d'au moins une autre sorte de diatomite pas ou peu calcinée ;
b. on remue le mélange dans environ 200 à 250 parts en poids, de préférence environ 225, d'eau en ajoutant 0,2 à 0,35 parts en poids, de préférence 0,27, d'au moins un produit de fluidification, et 0,4 à 0,8 parts en poids, de préférence 0,5 à 0,7, d'un liant organique ;
c. on remue le mélange au moins jusqu'à ce qu'il s'installe une nette modification de la viscosité, de préférence pendant environ 12 à 15 heures ;
d. une fois qu'on a fini de remuer le mélange, on le verse dans un moule (11, 13, 15) en un matériau absorbant l'humidité, de préférence du plâtre ;
e. on laisse sécher lentement le mélange, de préférence pendant 8 à 25 jours, notamment pendant 10 à 20 jours ;
f. on cuit le mélange séché, de préférence à une température maximale de 1.000 à 1.050 °C, notamment 1.035 °C.

12. Procédé selon la revendication 11, caractérisé en ce que l'on utilise les parts en poids suivantes :
environ 40,72 d'une première diatomite non calcinée ou peu calcinée,
environ 9,05 d'une seconde diatomite calcinée,
environ 20,82 d'une troisième diatomite non calcinée ou peu calcinée et
environ 29,41 de kaolin.

13. Procédé selon l'une des revendications 11 ou 12, caractérisé en ce que l'on ajoute au mélange 0,2 à 0,5 parts en poids, de préférence environ 0,36, d'oxyde d'argent.

14. Procédé selon l'une des revendications 11, 12 ou 13, caractérisé en ce que le moule (11, 13) est constitué d'un plâtre légèrement humide.

15. Procédé selon l'une des revendications 11 à 14, caractérisé en ce que le moule (11, 13, 15) comporte en son centre (14) un noyau (15) central en un matériau élastique et absorbant, de préférence un cylindre creux en papier ou en carton.

16. Procédé selon l'une des revendications 11 à 15, caractérisé en ce que, après la cuisson, on débarrasse la surface de la plaque, avec un outil usant, d'une couche extérieure serrée d'une épaisseur de 5 µm environ.

17. Procédé selon l'une des revendications 11 à 16, caractérisé en ce que l'on découpe aux dimensions exactes la plaque filtrante microporeuse cuite (1) au moyen d'un appareil à découper par jet d'eau à haute pression.

18. Procédé selon l'une des revendications 11 à 17, caractérisé en ce que l'on couvre le bord extérieur et le bord intérieur de la plaque avec des joints (5, 6, 7, 8) faisant le tour des bords sur au moins un côté, les joints étant de préférence à base de silicone.
